# EUROPÄISCHE PATENTSCHRIFT

(11) **EP 1 903 528 B1**
(45) Veröffentlichungstag und Bekanntmachung des Hinweises auf die Patenterteilung: **09.12.2009**
(21) Anmeldenummer: 07013848.2
(22) Anmeldetag: 14.07.2007
(51) Int. Cl.: G08B 29/14, G01N 33/00

(54) **Gasmeldeeinrichtung und Verfahren zum Überprüfen einer solchen**
Gas warning device and method for monitoring such
Dispositif d' alarme de gaz et procédé destiné à sa surveillance

(30) Priorität: 21.09.2006 DE 102006045055
(43) Veröffentlichungstag der Anmeldung: 26.03.2008
(73) Patentinhaber: Micronas GmbH, 79108 Freiburg i. Br. (DE); Hekatron Vertriebs GmbH, 79295 Sulzburg (DE)
(72) Erfinder: Lehmann, Mirko, Dr.rer.nat., 9642 Ebnat-Kappel (CH); Scherzinger, Hubert, 79108 Freiburg (DE)
(74) Vertreter: Huwer, Andreas

(56) Entgegenhaltungen:
- EP-A- 1 580 706
- DE-U1- 20 215 640
- US-A1- 2005 000 981

## Beschreibung

Die Erfindung betrifft eine Gasmeldeeinrichtung nach dem Oberbegriff von Anspruch 1 und ein Verfahren zum Überprüfen der Funktion einer mindestens zwei, in wenigstens einem zu überwachenden Raum angeordnete Gassensoren aufweisenden Gasmeldeeinrichtung nach dem Oberbegriff von Anspruch 15.

Eine derartige Gasmeldeeinrichtung ist aus EP 1 191 497 B1 bekannt. Sie weist mehrere, an der Decke eines Gebäudes angeordnete Brandmelder auf, in die jeweils ein Gassensor integriert ist. Die Gassensoren sind über eine zentrale Auswerteeinrichtung mit einem Alarmgeber verbunden. Zur Funktionsprüfung der Gassensoren ist eine mobile Testeinrichtung vorgesehen, die einen Prüftopf aufweist, der am Ende eines Haltestabs angeordnet ist. Mittels des Haltestabs kann der Prüftopf nacheinander unter den einzelnen Gassensoren positioniert werden. An dem Prüftopf ist ein Gasvorratsbehälter angeordnet, der mit einem Prüfgas befullt ist, für das die Gassensoren empfindlich sind. Der Gasvorratsbehälter ist über ein Absperrventil mit einer Gasauslassöffnung verbunden, die im Inneren des Prüftopf angeordnet ist. Zum Testen eines Gassensors wird die Gasmeldeeinrichtung zunächst in einen Prüfmodus versetzt. Dann wird der Prüftopf derart unter dem Gassensor positioniert, dass der Rand des Prüftopfs gegen die Decke abdichtet und den Gassensor umgrenzt. Danach wird das Absperrventil geöffnet, damit der Gassensor mit dem aus der Gasauslossöffnung austretenden Prüfgas in Kontakt gerät und ein Messsignal abgibt. Dieses wird von der Auswerteeinrichtung empfangen und interpretiert. Wenn das Messsignal ausbleibt, wird angenommen, dass der Gassensor defekt ist. Die Gasmeldeeinrichtung hat den Nachteil, dass das Testen der Gassensoren relativ zeitaufwändig und mühsam ist, da der Prüftopf während des Prüfvorganges nacheinander vom Wartungspersonal unter den einzelnen Gassensoren positioniert werden muss. Dabei kann es sein, dass die Gassensoren schlecht zugänglich sind, beispielsweise weil sie an einer hohen Gebäudedecke einer Lagerhalle angeordnet sind.

Aus DE 202 15 640 U1 ist ferner eine gattungsfremde Vorrichtung zum Prüfen von Branddetektoren und Brand- und Rauchschutzklappen bekannt. Die Vorrichtung weist mehrere Branddetektoren sowie Brand- und Rauchschutzklappen auf. Die Brand- und Rauchschutzklappen sollen beim Auftreten von Rauch in einem Gebäudeteil eine Verteilung des Rauchs in andere Gebäueteile vermeiden. Zur Überprüfung der Funktion der Brand- und Rauchschutzklappen hat die Vorrichtung eine Testeinrichtung, die einen Aerosolgenerator aufweist. Mit Hilfe des Aerosolgenerators wird ein Aerosol erzegt, das einen Branddetektor auslöst. Sobald der Branddetektor ausgelöst hat, wird die Brand- und Rauchschutzklappe betätigt. Die Zeit, die zum Betätigen der Brand- und Rauchschutzklappe nötig ist, wird von einer Zeitmesseinheit gemessen. Beim Überschreiten einer bestimten Zeitdauer zwischen Öffnen und Schließen wird eine Fehlermeldung generiert. In der DE 202 15 640 U1 istjedoch nicht beschrieben, wie der Aerosolgenerator relativ zu den Branddetektoren angeorndet ist.

Aufgabe der Erfindung ist es, eine Gasmeldeeinrichtung der eingangs genannten Art zu schaffen, die eine einfache, schnelle und zuverlässige Funktionsprüfung der Gassensoren ermöglicht. Ferner besteht die Aufgabe, ein Verfahren anzugeben, mit dem die Funktion eines Gassensors auf einfache Weise überprüft werden kann.

Diese Aufgabe wird gelöst durch eine Gasmeldeeinrichtung nach Anspruch 1.

Es wird also nur noch eine zentrale Gasauslassöffnung benötigt, mittels der sämtliche in dem Raum befindliche Gassensoren einer Funktionsüberprüfung unterzogen werden können. Die Gasauslassöffnung ist derart angeordnet und dimensioniert, dass der Gasauslassöfmung austretendes Prüfgas durch die in dem Raum befindliche Luft in einer Konzentration zu den Gassensoren diffundieren und/oder durch Konvektion gelangen kann, die von den Gassensoren sicher detektiert werden kann. Die Detektion des Prüfgases kann z.B. in der Weise erfolgen, dass die Messsignale der einzelnen Gassensoren jeweils mit einer Signalschwelle verglichen werden und dass bei Überschreiten der Signalschwelle die Anwesenheit des Prüfgases erkannt wird. Die Gasmeideeinrichtung kann als Brandmeldeanlage ausgestaltet oder in eine solche integriert sein. Dabei sind die Gassensoren jeweils für mindestens ein bei einem Entstehungsbrand und/oder einem Brand auftretendes Brandgas empfindlich. Die Brandmeldeanlage kann zusätzlich zu den Gassensoren mindestens einen optischen Rauchmelder aufweisen, der mit dem Gassensor in einem gemeinsamen Meldergehäuse angeordnet ist. Mittels der Testeinrichtung kann dann indirekt auch geprüft werden, ob der Rauchmelder von dem Prüfgas angeströmt wird (und somit auch von in dem zu überwachenden Raum befindlichem Rauch). Die Testeinrichtung kann auch bei der Erstinstallation einer Gasmeldeeinrichtung verwendet werden. Dabei ist es insbesondere möglich die Anordnung der Gassensoren im Raum während der Installation zu verändern, damit diese das Testgas besser detektieren können.

Bei einer bevorzugten Ausgestaltung der Erfindung ist die Gasauslassöffnung ortsfest zu dem Raum angeordnet. Die Gassensoren können dann noch einfacher und schneller auf ihre Funktion überprüft werden. Dabei ist es sogar möglich, die Gasmeldeeinrichtung Vollautomatisch zu testen, indem das Absperrventil mittels einer Ansteuereinrichtung in regelmäßigen Zeitabständen für eine vorbestimmte Zeitdauer automatisch geöffnet und überprüft wird, ob die Gassensoren das Prüfgas detektieren.

Zweckmäßigerweise ist die Gasauslassöffnung an einer Inerartgas-Löschanlage angeordnet, die zusätzlich zu dem das Testgas enthaltenden Gasvorratsbehälter einen Gasspeicher für ein Inertgas aufweist, der über eine Absperreinrichtung mit der Gasauslassöffnung verbunden ist Das Leitungsnetz und die Gasauslassöff nung(en) der Inerartgas-Löschanlage kann (können) dann wahlweise entweder zum Zuführen des Löschgases zu einer in dem zu überwachenden Raum befindlichen Brandstelle oder zum Zuführen des Testgases zu dem Gassensor bzw. den Gassensoren genutzt werden.

Bei einer anderen vorteilhaften Ausgestaltung der Erfindung sind der Gasvorratsbehälter, das Absperrventil und die Gasauslassöffnung als mobile Testeinheit ausgestaltet. Mit einer solchen mobilen Testeinheit können insbesondere in vorhandenen Räumen angeordnete oder zu installierende Gasmeldeeinrichtungen kostengünstig und schnell getestet werden, ohne dass Rohrleitungen für das Prüfgas verlegt werden müssen.

Vorteilhaft ist, wenn zwischen dem Gasvorratsbehälter und der Gasauslassöffnung ein Stellglied zum Einstellen des Prüfgas-Volumenstroms angeordnet ist und/oder das Absperrventil als Stellglied zum Einstellen des Volumenstroms ausgestaltet ist. Der Volumenstrom des freigesetzten Prüfgases kann dann während der Überprüfung der Gassensoren verändert werden, wodurch eine noch genauere Überprüfung des Ansprechverhaltens der Gassensoren möglich ist.

Bei einer bevorzugten Ausführungsform der Erfindung weist die Testeinrichtung einen Datenspeicher auf, in dem für jeden Gassensor ein Referenzwertbereich für die Laufzeit, die das Prüfgas für die Diffusion von der Gasauslassöffnung zu dem betreffenden Gassensor benötigt, abgelegt ist, wobei die Testeinrichtung eine Zeitmesseinrichtung zur Erfassung der Zeitdauern zwischen dem Öffnen des Absperrventils und der Detektion des Prüfgases an den einzelnen Gassensoren aufweist, und wobei die Testeinrichtung eine Vergleichsvorrichtung zum Vergleichen der gemessenen Zeitdauern mit den gespeicherten Referenzwertbereichen aufweist, die mit der Funktionsanzeige in Steuerverbindung steht. Dadurch ist es insbesondere möglich, Fehler zu detektieren, die durch ein verspätetes Ansprechen eines Gassensors auftreten können, was beispielsweise passieren kann, wenn der Gassensor von einem Gegenstand verdeckt oder zugestellt ist

Vorteilhaft ist, wenn in dem Datenspeicher der zeitliche Verlauf eines Test-Sollwertsignals für den mindestens einen Gassensor abgespeichert ist, und wenn die Testeinrichtung eine Vergleichseinrichtung zum Vergleichen des Test-Sollwertsignals mit dem zeitlichen Verlauf des Messsignals des mindestens einen Gassensors aufweist. Die Gasmeldeeinrichtung kann dann noch präziser auf ihre Funktion überprüft werden.

Zweckmäßigerweise ist die Gasmeldeeinrichtung zwischen einem Überwachungszustand, in dem die Auswerteeinrichtung mit dem Alarmgeber verbunden ist, und einem Testzustand umschaltbar, in dem ein Ausgang der Vergleichseinrichtung mit einer Funktionsanzeige für den mindestens einen Gassensor verbunden ist. Dadurch wird während der Funktionsüberprüfung das Auslösen eines Gasalarms vermieden. Die Umschaltung zwischen dem Überwachungszustand und dem Testzustand kann ggf, automatisch in vorgegebenen Zeitzyklen erfolgen. Dazu kann die Anstiegsgeschwindigkeit der Prüfgaskonzentration gemessen und mit einem Grenzwert verglichen vererden. Beim Überschreiten des Grenzwerts wird der Testzustand bzw. ein Prüfzyklus aktiviert.

Bei einer vorteilhaften Ausgestaltung der Erfindung sind mindestens drei Gassensoren in unterschiedlichen Räumen eines Gebäudes oder eines Fahrzeugs vorgesehen, wobei in jedem dieser Räume jeweils mindestens eine Gasauslassöffnung ortsfest zu dem betreffenden Raum angeordnet ist, und wobei der Gasvorratsbehälter als zentraler, über das zumindest eine Absperrventil und mindestens eine Prüfgas-Leitung mit den Gasauslassöffnungen verbundener Gasvorratsbehälter ausgestaltet ist. Die Handhabung der Gasmeldeeinrichtung ist dann noch komfortabler, da der Füllstand des Prüfgasvorrats zentral kontrolliert und ggf. aufgefüllt werden kann. Das Fahrzeug kann z.B. ein Schiff, ein Flugzeug, ein Schienenfahrzeug oder ein Kraftfahrzeug (PKW, LKW, Wohnwagen etc.) sein.

Vorteilhaft ist, wenn die Gasmeldeeinrichtung eine Ansteuereinrichtung für das mindestens eine Absperrventil aufweist, mittels der das Absperrventil derart ansteuerbar ist, dass an der Gasauslassöffnung ein sich von einem im Überwachungszustand auftretenden Gas-Konzentrationsmuster unterscheidendes Prüfgas-Konzentrationsmuster abgegeben wird, wobei der mindestens eine Gassensor eine Detektionseinrichtung zum Detektieren des Prüfgas-Konzentrationsmusters aufweist, und wobei beim Detektieren des Prüfgas-Konzentrationsmusters die Funktionsanzeige aktiviert wird. Ein durch das Prüfgas ausgelöster Alarm kann dann auf einfache Weise von einem "echten" Alarm unterschieden werden. Dadurch ist es insbesondere möglich, das Prüfgas mit einer größeren Empfindlichkeit zu detektieren, als das "echte" Gas, so dass die Funktionsüberprüfung mit einer entsprechend reduzierten Prüfgaskonzentration durchgeführt werden kann. Dadurch wird einerseits Prüfgas eingespart und andererseits kann an in dem zu überwachenden Raum befindlichen Arbeitsplätzen die maximal zulässige Prüfgas bzw. Arbeitsplatzkonzentration leichter eingehalten werden.

Das Prüfgas ist bevorzugt nicht toxisch. Bei einer Gasmeldeeinrichtung, die zur Detektion eines Brandgases vorgesehen ist, kann das Prüfgas beispielsweise Wasserstoff sein, das vorzugsweise bei einem Schwelbrand generell mit Kohlenmonoxid zusammen auftritt (Verhältnis H₂/CO z.B etwa 1 /3). Das Prüfgas kann aber auch ein Gas sein, das bei einem Brand normalerweise nicht auftritt und für das der Gassensor eine Querempfindlichkeit aufweist. Es ist aber auch möglich, dass der Gassensor bei seiner Konstruktion so ausgestaltet wird, dass er für ein nicht toxisches und vorzugsweise nicht brennbares Prüfgas empfindlich ist, beispielsweise indem bei einem Suspended-Gate-Feldeffekttransistor eine zusätzliche gassensitive Schicht für dieses Prüfgas eingebaut wird. Das Prüfgas kann aber auch ein toxisches Gas sein, sofern die Gasauslassöffnung so dimensioniert und auf den Prüfgasdruck und den zu überwachenden Raum abgestimmt ist, dass die Konzentration des Prüfgases in dem Raum die maximal zulässige Arbeitplatzkonzentration (MAK) nicht überschreitet, insbesondere eine Konzentration von 50 ppm und bevorzugt von 30 ppm.

Zweckmäßigerweise ist benachbart zu jedem Gassensor ein Temperatursensor zur Erfassung der Umgebungstemperatur des Gassensors angeordnet ist. Dadurch ist es möglich, bei der Funktionsüberprüfung den Einfluss der Umgebungstemperatur auf die Diffusion und/oder Konvektion des Prüfgases in dem zu überwachenden Raum zu berücksichtigen.

Vorteilhaft ist, wenn die Gasmeldeeinrichtung zum Temperieren des Testgases eine Heizung und/oder Kühlvorrichtung aufweist. Bei der Funktionsüberprüfung der Gassensoren kann dann gleichzeitig auch die Funktion des Temperatursensors überprüft werden. Außerdem kann überprüft werden, ob die Gasmeldeeinrichtung auch heiße Gase, die z.B. auch bei einem Brand auftreten können, zuverlässig detektieren kann.

Das Prüfgas kann ein Gasgemisch sein, das zumindest ein erstes und ein zweites Gas enthält, die in Luft eine unterschiedliche Diffusionsgeschwindigkeit aufweisen, wobei für das erste Gas mindestens ein erster Gassensor und für das zweite Gas mindestens ein zweiter Gassensor empfindlich ist, und wobei der erste Gassensor und der zweite Gassensor in einem gemeinsamen Meldergehäuse angeordnet sind. Bei der Funktionsüberprüfung kann dann untersucht werden, welchen Einfluss die Diffusionsgeschwindigkeit der in dem Prüfgas enthaltenen Gase auf das Detektionsverhalten des Melders hat. Das Prüfgas kann z.B. Wasserstoff und Ammoniak enthalten. Dabei hat der Wasserstoff wegen seiner relativ kleinen Moleküle eine größere Diffusionsgeschwindigkeit als das Ammoniak.

Die vorstehend genannte Aufgabe wird bezüglich eines Verfahrens zum Überprüfen der Funktion einer mindestens zwei, in wenigstens einem zu überwachenden Raum angeordneten Gassensoren aufweisenden Gasmeldeeinrichtung, wobei ein Prüfgas freigesetzt wird, für das die Gassensoren empfindlich sind, und wobei das Prüfgas mit Hilfe der Gassensoren detektiert wird, dadurch gelöst, dass Prüfgas derart an einer Stelle des Raums freigesetzt wird, dass es von dieser Stelle zu den mindestens zwei Gassensoren gelangt und von jedem dieser Gassensoren jeweils detektiert wird.

In vorteilhafter Weise kann dabei das Prüfgas mittels einer zentralen Gasauslassöffnung mehreren Gassensoren gleichzeitig in einer Konzentration zugeführt werden, die mit den Gassensoren detektiert werden kann. Somit können diese einfach und schnell auf ihre Funktion überprüft werden.

Bei einer bevorzugten Ausführungsform der Erfindung wird die Zeitdauer zwischen dem Freisetzen des Prüfgases und dem Detektieren des Prüfgases gemessen und mit einem Referenzwertbereich verglichen, wobei beim Auftreten einer Abweichung zwischen der gemessenen Zeitdauer und dem Referenzwertbereich ein Fehler detektiert wird. Dadurch lässt sich insbesondere feststellen, ob ein Gassensor beispielsweise durch einen in dem zu überwachenden Raum abgestellten Gegenstand verdeckt ist und daher das Prüfgas erst mit einer Verzögerung detektiert. Eine solche Verzögerung wird als Fehler gewertet. Wenn ein Gassensor nach einer vorgegebenen Messdauer kein Signal abgibt, wird die Messung der Zeitdauer abgebrochen und der betreffende Gassensor als defekt erkannt.

Bei einer vorteilhaften Ausgestaltung der Erfindung ist vorgesehen, dass das Prüfgas zunächst an einer ersten Stelle des zu überwachenden Raums freigesetzt und danach mit Hilfe der Gassensoren detektiert wird, dass dann das Prüfgas aus dem Raum entfernt wird, und dass das Prüfgas danach an einer zweiten Stelle des zu überwachenden Raums erneut freigesetzt und mit Hilfe der Gassensoren detektiert wird. Der Ort, an dem das Prüfgas freigesetzt wird, kann also auch variiert werden.
Dadurch kann die Gasmeldeeinrichtung noch gründlicher getestet werden.

Nachfolgend ist ein Ausführungsbeispiel der Erfindung anhand der Zeichnung näher erläutert. Es zeigt
- Fig. 1: eine schematische Darstellung eines ersten Ausführungsbeispiels einer Gasmeldeeinrichtung, die mehrere in einem Gebäude verteilt angeordnete Gassensoren aufweist,
- Fig. 2: eine graphische Darstellung der einzelnen Messsignale der Gassensoren während einer Funktionsüberprüfung der Gasmeldeeinrichtung, wobei auf der Abszisse die Zeit und auf der Ordinate die Amplitude der Messsignale aufgetragen ist,
- Fig. 3: eine graphische Darstellung von Meldesignalen der Gassensoren, die abgegeben werden, wenn das Messsignal eine vorbestimmte Signalschwelle überschreitet, wobei auf der Abszisse die Zeit und auf der Ordinate die Amplitude der Meldesignale aufgetragen ist, und
- Fig. 4: eine schematische Darstellung eines zweiten Ausführungsbeispiels einer Gasmeldeeinrichtung, die mit einer Inertgas-Löschanlage kombiniert ist.

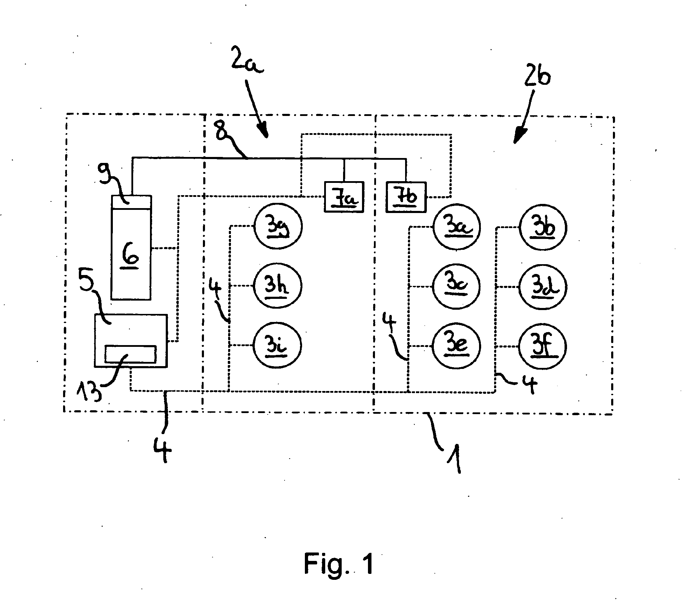

Eine in einem Gebäude 1 oder einem Fahrzeug mit mehreren zu überwachenden, durch Wandungen begrenzten Räumen 2a, 2b angeordnete Casmeldeeinrichtung einer Brandmeldeanlage hat in jedem zu überwachenden Raum 2a, 2b jeweils mehrere voneinander beabstandete Gassensoren 3a - 3i, die für ein Brandgas, wie z.B. Kohlenmonoxid empfindlich sind. Wie in Fig. 1 erkennbar ist, sind die Gassensoren 3a - 3i über Signalleitungen 4 mit einer zentralen Auswerteeinrichtung 5 verbunden, die als Brandmeldezentrale mit einem nicht näher dargestellten Alarmgeber ausgestaltet ist. Die Auswerteeinrichtung 5 kann einen Mikrocomputer mit einem Mikroprozessor, einem Datenspeicher 13 und Ein-Ausgabegeräten, wie z.B. einer Anzeigeinrichtung, aufweisen. Mehrere Gassensoren 3a - 3i oder Gruppen von Gassensoren 3a - 3i können an einer gemeinsamen Melderlinie angeschlossen sein, die direkt oder indirekt mit der Auswerteeinrichtung 5 verbunden ist. Die Melderlinie kann in an sich bekannter Weise mit einem elektronischen Bussystem ausgestattet sein, das die Gassensoren miteinander verbindet.

Zur Funktionsprüfung der Gassensoren 3a - 3i ist eine Testeinrichtung vorgesehen, die fest in dem Gebäude 1 installiert ist. Die Testeinrichtung weist einen zentralen Gasvorratsbehälter 6 auf, der mit einem Prüfgas befüllt ist, für das die Gassensoren 3a - 3i empfindlich sind. In jedem zu überwachenden Raum 2a, 2b des Gebäudes 1 ist jeweils eine Gasauslassöffnung 7a, 7b vorgesehen, die von den Gassensoren 3a - 3i beabstandet und ortsfest zu diesen angeordnet ist, beispielsweise an der Decke des Raums 2a, 2b. Die Gasauslassöffnungen 7a, 7b sind über Prüfgas-Leitungen 8, die z.B. Rohre und/oder Schläuche umfassen können, an dem Gasvorratsbehälter 6 angeschlossen. Zwischen dem Gasvorratsbehälter 6 und den Gasauslassöffnungen 7a, 7b ist mindestens ein mit der Auswerteeinrichtung 5 in Steuerverbindung stehendes Absperrventil 9 angeordnet, mit dem der Prüfgasstrom zu den Gasauslassöffnungen 7a, 7b in Abhängigkeit von einem Steuersignal, das in der Auswerteeinrichtung 5 erzeugt wird, gesperrt oder freigegeben werden kann. Der Druck des Prüfgases, die Durchlassquerschnitte des Absperrventils 9, der Prüfgas-Leitungen 8 und der Gasauslassöffnungen 7a, 7b sowie die Abstände zwischen den Gasauslassöffnungen 7a, 7b und den Gassensoren 3a - 3i sind derart auf die Messempfindlichkeit der Gassensoren 3a - 3i abgestimmt, dass aus den Gasauslassöffnungen 7a, 7b austretendes Testgas durch die in den Räumen 2a, 2b befindliche Luft in einer Konzentration zu den Gassensoren 3a - 3i diffundieren kann, die bei ordnungsgemäßer Funktion der Gasmeldeeinrichtung an den einzelnen Gassensoren 3a - 3i jeweils eine Messsignaländerung verursacht.

Die Gasmeldeeinrichtung ist zwischen einem Überwachungszustand und einem Testzustand umschaltbar. In dem Überwachungszustand ist das Absperrventil 9 geschlossen und die Auswerteeinrichtung ist mit dem Alarmgeber verbunden und aktiviert diesen, wenn das Messsignal mindestens eines Gassensors 3a - 3i einen vorgegebenen Grenzwert überschreitet. Dabei kann gegebenenfalls eine Plausibilitätsprüfung durchgeführt werden, bei der ein Alarm nur dann ausgelöst wird, wenn an mehreren Gassensoren 3a - 3i der Grenzwert überschritten wird.

In dem Testzustand ist das Absperrventil 9 geöffnet. Die Ansteuerung des Alarmgebers ist gesperrt und die Auswerteeinrichtung 5 ist mit einer Funktionsanzeige für die Gassensoren 3a - 3i verbunden. Zu Beginn des Testvorgangs wird das Absperrventil 9 für eine vorgegebene Zeitdauer geöffnet, so dass Prüfgas aus dem Gasvorratsbehälter 6 in die zu überwachenden Räume 2a, 2b ausströmen und von den Gasauslassöffnungen 7a, 7b zu den Gassensoren 3a - 3i diffundieren kann.

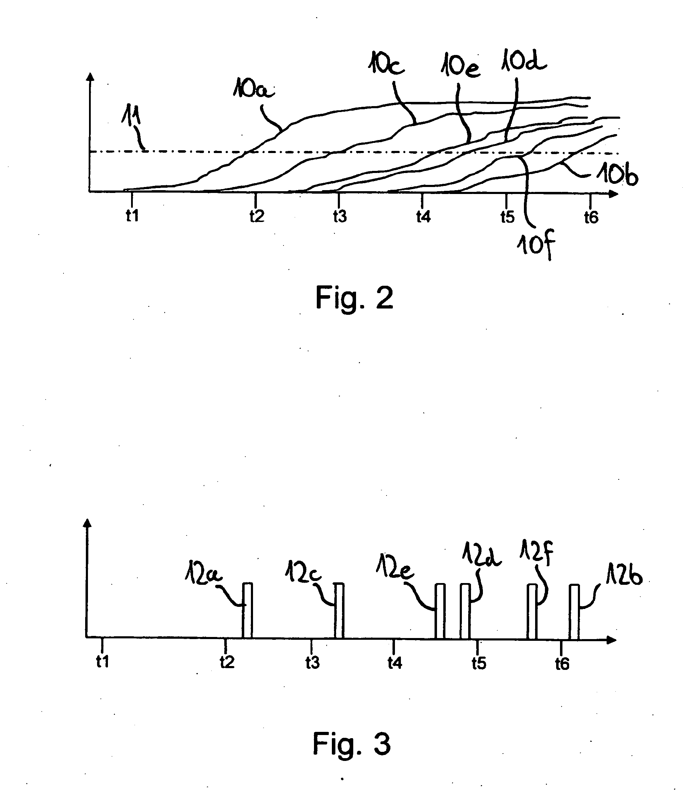

In Fig. 2 ist sind die Messsignale 10a-10i der im Raum 2b befindlichen Gassensoren 3a - 3f graphisch dargestellt. Dabei ist das Messsignal 10a dem Gassensor 3a, das Messsignal 10b dem Gassensor 3b, das Messsignal 10c ist dem Gassensor 3c zugeordnet usw. Der Zeitpunkt, an dem die Gasauslassöffnung 7b geöffnet wird, ist auf der Abszisse mit t1 bezeichnet.

Deutlich ist erkennbar, dass an dem am nächsten zu der Gasauslassöffnung 7b angeordneten Gassensor 3a früherer ein Messsignalanstieg detektiert wird als an den weiter von der Gasauslassöffnung 7b entfernten Gassensoren 3b - 3f Außerdem nimmt die Anstiegsgeschwindigkeit der Messsignale mit zunehmendem Abstand der Gassensoren 3a - 3f von der Gasauslassöffnung 7b langsam ab, da sich die Prüfgaskonzentration in dem Raum 2b mit zunehmendem Abstand von der Gasauslassöffnung 7b reduziert.

In Fig. 2 und 3 ist erkennbar, dass die einzelnen Messsignale 10a-10f jeweils mit einer vorgegeben Signalschwelle 11 verglichen werden, und dass beim Überschreiten der Signalschwelle 11 jeweils ein Meldungssignal 12a-12f erzeugt wird. Das Meldungssignal 12a ist dem Gassensor 12a, das Meldungssignal 12b dem Gassensor 3b zugeordnet usw. Die Meldungssignale 12a-12f treten in unterschiedlichen Zeitfenstern auf, nämlich das Meldungssignal 12a in dem Zeitfenster t2 - t3, das Meldungssignal 12c in dem Zeitfenster t3 - t4, die Meldungssignale 12e und 12d in dem Zeitfenster t4 - t5, das Meldungssignal 12f in dem Zeitfenster t5 - t6 und das Meldungssignal 12b in dem Zeitfenster nach t6. Die Meldungssignale 12a-12f werden über die Signalleitungen 4 an die Auswerteeinrichtung 5 übermittelt.

In dem Datenspeicher 13 ist für jeden Gassensor 3a-3i jeweils ein Zeitfester als Referenzwertbereich für die Laufzeit, die das Prüfgas für die Diffusion von der Gasauslassöffnung 7a, 7b zu dem betreffenden Gassensor 3a-3i bei ordnungsgemäßer Funktion der Gasmeldeeinrichtung benötigt, abgelegt. Zur Erfassung der Zeitdauern zwischen dem Öffnen des Absperrventils 9 und der Detektion des Prüfgases an den einzelnen Gassensoren 3a-3i weist die Testeinrichtung eine in der Zeichnung nicht näher dargestellte Zeitmesseinrichtung auf Die gemessenen Zeitdauern werden mit den Referenzwertbereichen verglichen. Wenn bei allen Gassensoren 3a-3i die Zeitdauer jeweils innerhalb des betreffenden Referenzwertbereichs liegt, wird an der Funktionsanzeige die Funktionsbereitschaft der Gasmeldeeinrichtung angezeigt. Wenn bei mindestens einem Gassensor 3a-3i die gemessene Zeitdauer außerhalb des Referenzwertbereichs liegt, wird an der Funktionsanzeige eine Fehlermeldung angezeigt, in welcher der mindestens eine defekte Gassensor 3a-3i aufgeführt ist. Dieser muss dann ausgetauscht oder repariert werden und/oder es müssen Gegenstände, die vor dem als defekt ausgewiesenen Gassensor 3a-3i abgestellt sind, entfernt werden.

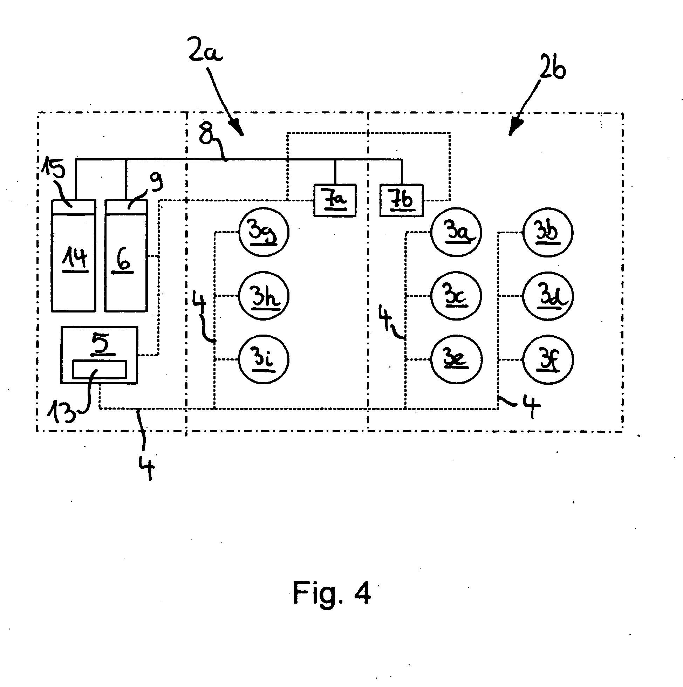

Bei dem in Fig. 4 gezeigten Ausführungsbeispiel ist die Gasmeldeeinrichtung mit einer Inertgas-Löschanlage kombiniert. Die Inertgas-Löschanlage hat einen Gasspeicher 14, der mit einem Inertgas, wie z.B. Kohlendioxid oder Stickstoff befüllt ist. Der Gasspeicher 14 ist über eine Absperreinrichtung mit Leitungen 8 verbunden, die zu den Gasauslassöffnungen 7a, 7b führen. Außerdem sind die Leitungen 8 über das Absperrventil an dem Gasvorratsbehälter 6 angeordnet. Somit kann über die Leitungen 8 wahlweise das Testgas oder das Inertgas zu den Gasauslassöffnungen 7a, 7b geleitet werden. Im Übrigen entspricht das in Fig. 4 gezeigte Ausführungsbeispiel dem Ausführungsbeispiel nach Fig. 1, weshalb das zu Fig. 1 Gesagte für Fig. 4 entsprechend gilt.

## Patentansprüche

1. Gasmeldeeinrichtung mit mindestens zwei, in wenigstens einem zu überwachenden Raum (2a, 2b) angeordneten Gassensoren (3a - 3i), die über eine Auswerteeinrichtung (5) mit einem Alarmgeber verbunden sind, und mit einer Testeinrichtung zur Funktionsprüfung der Gassensoren (3a - 3i), wobei die Testeinrichtung einen Gasvorratsbehälter (6) aufweist, in dem ein Prüfgas angeordnet ist, für das die Gassensoren (3a - 3i) empfindlich sind, und wobei der Gasvorratsbehälter (6) über zumindest ein Absperrventil (9) mit mindestens einer Gasauslassöffnung (7a, 7b) verbunden ist, **dadurch gekennzeichnet, dass** die Gasauslassöffnung (7a, 7b) derart ausgestaltet und relativ zu den Gassensoren (3a - 3i) angeordnet ist, dass aus ihr austretendes Testgas zu mindestens zwei Gassensoren (3a - 3i) gelangen und an diesen jeweils ein Messsignal (10a -10f) verursachen kann.

2. Gasmeldeeinrichtung nach Anspruch 1, **dadurch gekennzeichnet, dass** die Gasauslassöffnung (7a, 7b) ortsfest zu dem Raum (2a, 2b) angeordnet ist.

3. Gasmeldeeinrichtung nach Anspruch 1 oder 2, **dadurch gekennzeichnet**, die Gasauslassöffnung (7a, 7b) an einer Inerartgas-Löschanlage angeordnet ist, die zusätzlich zu dem das Testgas enthaltenden Gasvorratsbehälter (6) einen Gasspeicher (14) für ein Inertgas aufweist, der über eine Absperreinrichtung (15) mit der Gasauslassöffnung (7a, 7b) verbunden ist.

4. Gasmeldeeinrichtung nach einem der Ansprüche 1 bis 3, **dadurch gekennzeichnet, dass** der Gasvorratsbehälter (6), das Absperrventil (9) und die Gasauslossöffnung (7a, 7b) als mobile Testeinheit ausgestaltet ist.

5. Gasmeideeinrichtung nach einem der Ansprüche 1 bis 4, **dadurch gekennzeichnet, dass** zwischen dem Gasvorratsbehälter (6) und der Gasauslassöffnung (7a, 7b) ein Stellglied zum Einstellen des Prüfgas-Volumenstroms angeordnet ist und/oder dass das Absperrventil (9) als Stellglied zum Einstellen des Volumenstroms ausgestaltet ist.

6. Gasmeldeeinrichtung nach einem der Ansprüche 1 bis 5, **dadurch gekennzeichnet, dass** die Testeinrichtung einen Datenspeicher (13) aufweist in dem für jeden Gassensor (3a - 3i) einen Referenzwertbereich für die Laufzeit, die das Prüfgas für die Diffusion von der Gasauslassöffnung (7a, 7b) zu dem betreffenden Gassensor (3a - 3i) benötigt, abgelegt ist, dass die Testeinrichtung eine Zeitmesseinrichtung zur Erfassung der Zeitdauern zwischen dem Öffnen des Absperrventils (9) und der Detektion des Prüfgases an den einzelnen Gassensoren (3a - 3i) aufweist, und dass die Testeinrichtung eine Vergleichsvorrichtung zum Vergleichen der gemessenen Zeitdauern mit den gespeicherten Referenzwertbereichen aufweist, die mit der Funktionsanzeige in Steuerverbindung steht.

7. Gasmeldeeinrichtung nach einem der Ansprüche 1 bis 6, **dadurch gekennzeichnet, dass** in dem Datenspeicher (13) der zeitliche Verlauf eines Test-Sollwertsignals für den mindestens einen Gassensor (3a - 3i) abgespeichert Ist, und dass die Testeinrichtung eine Vergleichseinrichtung zum Vergleichen des Test-Sollwertsignals mit dem zeitlichen Verlauf des Messsignals (10a -10f) des mindestens einen Gassensors (3a - 3i) aufweist

8. Gasmeldeeinrichtung nach einem der Ansprüche 1 bis 7, **dadurch gekennzeichnet, dass** sie zwischen einem Überwachungszustand, in dem die Auswerteeinrichtung (5) mit dem Alarmgeber verbunden ist, und einem Testzustand umschaltbar ist in dem ein Ausgang der Vergleichseinrichtung mit einer Funktionsanzeige für den mindestens einen Gassensor (3a - 3i) verbunden ist.

9. Gasmeideeinrichtung nach einem der Ansprüche 1 bis 8, **dadurch gekennzeichnet, dass** mindestens drei Gassensoren (3a - 3i) in unterschiedlichen Räumen (2a, 2b) eines Gebäudes (1) oder eines Fahrzeugs vorgesehen sind, dass in jedem dieser Räume (2a, 2b) jeweils mindestens eine Gasauslassöffnung (7a, 7b) ortsfest zu dem betreffenden Raum (2a, 2b) angeordnet ist, und dass der Gasvorratsbehälter (6) als zentraler, über das zumindest eine Absperrventil (9) und mindestens eine Prüfgas-Leitung (8) mit den Gasauslassöffnungen (7a, 7b) verbundener Gasvorratsbehälter (6) ausgestattet ist.

10. Casmeldeeinrichtung nach einem der Ansprüche 1 bis 9, **dadurch gekennzeichnet, dass** sie eine Ansteuereinrichtung für das mindestens eine Absperrventil (9) aufweist, mittels der das Absperrventil (9) derart ansteuerbar ist, dass an der Casauslassöffnung (7a, 7b) ein sich von einem im Überwachungszustand auftretenden Gas-Konzentrationsmuster unterscheidendes Prüfgas-Konzentrdtionsmuster abgegeben wird, dass der mindestens eine Gassensor (3a - 3i) eine Detektionseinrichtung zum Detektieren des Prüfgas-Konzentrationsmusters aufweist, und dass beim Detektieren des Prüfgas-Konzentrationsmusters die Funktionsanzeige aktiviert wird.

11. Gasmeldeeinrichtung nach einem der Ansprüche 1 bis 10, **dadurch gekennzeichnet, dass** das Prüfgas nicht toxisch ist.

12. Gasmeldeeinrichtung nach einem der Ansprüche 1 bis 11, **dadurch gekennzeichnet, dass** benachbart zu jedem Gassensor (3a - 3i) ein Temperatursensor zur Erfassung der Umgebungstemperatur des Gassensors (3a - 3i) angeordnet ist

13. Gasmeldeeinrichtung nach einem der Ansprüche 1 bis 12, **dadurch gekennzeichnet, dass** die Gasmeldeeinrichtung zum Temperieren des Testgases eine Heizung und/oder Kühlvorrichtung aufweist.

14. Gasmeldeeinrichtung nach einem der Ansprüche 1 bis 1 3, **dadurch gekennzeichnet, dass** das Prüfgas ein Gasgemisch ist, das zumindest ein erstes und ein zweites Gas enthält, die in Luft eine unterschiedliche Diffusionsgeschwindigkeit aufweisen, dass für das erste Gas mindestens ein erster Gassensor (3a - 3i) und für das zweite Gas mindestens ein zweiter Gassensor (3a - 3i) empfindlich ist, und dass der erste Gassensor (3a - 3i) und der zweite Gassensor (3a - 3i) in einem gemeinsamen Meldergehäuse angeordnet sind.

15. Verfahren zum Überprüfen der Funktion einer mindestens zwei, in wenigstens einem zu überwachenden Raum (2a, 2b) angeordneten Gassensoren (3a - 3i) aufweisenden Gasmeideeinrichtung, wobei ein Prüfgas freigesetzt wird, für das die Gassensoren (3a - 3i) empfindlich sind, und wobei das Prüfgas mit Hilfe der Gassensoren (3a - 3i) detektiert wird, **dadurch gekennzeichnet, dass** Prüfgas derart an einer Stelle des Raums freigesetzt wird, dass es von dieser Stelle zu den mindestens zwei Gassensoren (3a - 3i) gelangt und von jedem dieser Gassensoren jeweils detektiert wird.

16. Verfahren nach Anspruch 15, **dadurch gekennzeichnet, dass** die Zeitdauer zwischen dem Freisetzen des Prüfgases und dem Detektieren des Prüfgases gemessen und mit einem Referenzwertbereich verglichen wird, und dass beim Auftreten einer Abweichung zwischen der gemessenen Zeitdauer und dem Referenzwertbereich ein Fehler detektiert wird.

17. Verfahren nach Anspruch 15 oder 16, **dadurch gekennzeichnet dass** das Prüfgas an einer ersten Stelle des zu überwachenden Raums freigesetzt und danach mit Hilfe der Gassensoren detektiert wird, dass dann das Prüfgas aus dem Raum entfernt wird, und dass das Prüfgas danach an einer zweiten Stelle des zu überwachenden Raums erneut freigesetzt und mit Hilfe der Gassensoren detektiert wird.

18. Verfahren nach einem der Ansprüche 15 bis 17, **dadurch gekennzeichnet, dass** das Prüfgas ein Gasgemisch ist, das mindestens zwei Gase enthält, die in Luft eine unterschiedliche Diffusionsgeschwindigkeit aufweisen.

## Claims

1. Gas detector having at least two gas sensors (3a - 3i) which are arranged in at least one room (2a, 2b) to be monitored and are connected to an alarm transmitter via an evaluation device (5), and having a test device for functionally testing the gas sensors (3a - 3i), the test device having a gas storage container (6) in which a test gas to which the gas sensors (3a - 3i) are sensitive is arranged, and the gas storage container (6) being connected to at least one gas outlet opening (7a, 7b) via at least one shut-off valve (9), **characterized in that** the gas outlet opening (7a, 7b) is configured and arranged relative to the gas sensors (3a - 3i) in such a manner that test gas emerging from it can pass to at least two gas sensors (3a - 3i) and can give rise to a respective measurement signal (10a - 10f) at said sensors.

2. Gas detector according to Claim 1, **characterized in that** the gas outlet opening (7a, 7b) is arranged such that it is stationary with respect to the room (2a, 2b).

3. Gas detector according to Claim 1 or 2, **characterized in that** the gas outlet opening (7a, 7b) is arranged on an inert gas extinguishing system which, in addition to the gas storage container (6) containing the test gas, has a gas store (14) for an inert gas, which gas store is connected to the gas outlet opening (7a, 7b) via a shut-off device (15).

4. Gas detector according to one of Claims 1 to 3, **characterized in that** the gas storage container (6), the shut-off valve (9) and the gas outlet opening (7a, 7b) are in the form of a mobile test unit.

5. Gas detector according to one of Claims 1 to 4, **characterized in that** an actuator for setting the volumetric flow of test gas is arranged between the gas storage container (6) and the gas outlet opening (7a, 7b), and/or **in that** the shut-off valve (9) is in the form of an actuator for setting the volumetric flow.

6. Gas detector according to one of Claims 1 to 5, **characterized in that** the test device has a data memory (13) which stores, for each gas sensor (3a - 3i), a reference value range for the transit time needed by the test gas to diffuse from the gas outlet opening (7a, 7b) to the relevant gas sensor (3a - 3i), **in that** the test device has a time measuring device for detecting the periods of time between the opening of the shut-off valve (9) and the detection of the test gas at the individual gas sensors (3a - 3i), and **in that** the test device has a comparison apparatus for comparing the measured periods of time with the stored reference value ranges, which comparison apparatus has a control connection to the function display.

7. Gas detector according to one of Claims 1 to 6, **characterized in that** the data memory (13) stores the temporal profile of a test desired value signal for the at least one gas sensor (3a - 3i), and **in that** the test device has a comparison device for comparing the test desired value signal with the temporal profile of the measurement signal (10a - 10f) from the at least one gas sensor (3a - 3i).

8. Gas detector according to one of Claims 1 to 7, **characterized in that** it can be changed over between a monitoring state, in which the evaluation device (5) is connected to the alarm transmitter, and a test state in which an output of the comparison device is connected to a function display for the at least one gas sensor (3a - 3i).

9. Gas detector according to one of Claims 1 to 8, **characterized in that** at least three gas sensors (3a - 3i) are provided in different rooms (2a, 2b) of a building (1) or spaces of a vehicle, **in that** at least one gas outlet opening (7a, 7b) is respectively arranged in each of these rooms/spaces (2a, 2b) in such a manner that it is stationary with respect to the relevant room/space (2a, 2b), and **in that** the gas storage container (6) is in the form of a central gas storage container (6) which is connected to the gas outlet openings (7a, 7b) via the at least one shut-off valve (9) and at least one test gas line (8).

10. Gas detector according to one of Claims 1 to 9, **characterized in that** it has a drive device for the at least one shut-off valve (9), which drive device can be used to drive the shut-off valve (9) in such a manner that a test gas concentration pattern which differs from a gas concentration pattern in the monitoring state is output at the gas outlet opening (7a, 7b), **in that** the at least one gas sensor (3a - 3i) has a detection device for detecting the test gas concentration pattern, and **in that** the function display is activated when detecting the test gas concentration pattern.

11. Gas detector according to one of Claims 1 to 10, **characterized in that** the test gas is non-toxic.

12. Gas detector according to one of Claims 1 to 11, **characterized in that** a temperature sensor for detecting the ambient temperature of the gas sensor (3a - 3i) is arranged adjacent to each gas sensor (3a - 3i).

13. Gas detector according to one of Claims 1 to 12, **characterized in that** the gas detector has a heater and/or cooling apparatus for bringing the test gas to the correct temperature.

14. Gas detector according to one of Claims 1 to 13, **characterized in that** the test gas is a gas mixture containing at least one first gas and one second gas which have different diffusion rates in air, **in that** at least one first gas sensor (3a - 3i) is sensitive to the first gas and at least one second gas sensor (3a - 3i) is sensitive to the second gas, and **in that** the first gas sensor (3a - 3i) and the second gas sensor (3a - 3i) are arranged in a common detector housing.

15. Method for checking the function of a gas detector having at least two gas sensors (3a - 3i) arranged in at least one room (2a, 2b) to be monitored, a test gas to which the gas sensors (3a - 3i) are sensitive being released, and the test gas being detected with the aid of the gas sensors (3a - 3i), **characterized in that** test gas is released at a point in the room in such a manner that it passes from this point to the at least two gas sensors (3a - 3i) and is respectively detected by each of these gas sensors.

16. Method according to Claim 15, **characterized in that** the period of time between the release of the test gas and the detection of the test gas is measured and is compared with a reference value range, and **in that** an error is detected in the event of a difference between the measured period of time and the reference value range.

17. Method according to Claim 15 or 16, **characterized in that** the test gas is released at a first point in the room to be monitored and is then detected with the aid of the gas sensors, **in that** the test gas is then removed from the room, and **in that** the test gas is then released again at a second point in the room to be monitored and is detected with the aid of the gas sensors.

18. Method according to one of Claims 15 to 17, **characterized in that** the test gas is a gas mixture containing at least two gases which have different diffusion rates in air.

## Revendications

1. Dispositif de détection de gaz comprenant au moins deux détecteurs de gaz (3a - 3i) disposés dans au moins un espace à surveiller (2a, 2b), lesquels sont reliés avec un émetteur d'alarme par le biais d'un dispositif d'interprétation (5), et comprenant un dispositif de test pour vérifier le fonctionnement des détecteurs de gaz (3a - 3i), le dispositif de test présentant un réservoir de gaz (6) dans lequel se trouve un gaz témoin auquel les détecteurs de gaz (3a - 3i) sont sensibles, et le réservoir de gaz (6) étant relié avec au moins une ouverture de sortie de gaz (7a, 7b) par le biais d'au moins une vanne d'arrêt (9), **caractérisé en ce que** l'ouverture de sortie de gaz (7a, 7b) est configurée et disposée par rapport aux détecteurs de gaz (3a - 3i) de telle sorte que le gaz témoin qui s'en échappe atteint au moins deux détecteurs de gaz (3a - 3i) et peut à chaque fois produire un signal de mesure (10a - 10f) au niveau de ceux-ci.

2. Dispositif de détection de gaz selon la revendication 1, **caractérisé en ce que** l'ouverture de sortie de gaz (7a, 7b) est disposée en position fixe par rapport à l'espace (2a, 2b).

3. Dispositif de détection de gaz selon la revendication 1 ou 2, **caractérisé en ce que** l'ouverture de sortie de gaz (7a, 7b) est disposée sur un équipement d'extinction à gaz inerte qui présente, en plus du réservoir de gaz (6) contenant le gaz témoin, un accumulateur de gaz (14) pour un gaz inerte qui est relié avec l'ouverture de sortie de gaz (7a, 7b) par le biais d'un dispositif d'arrêt (15).

4. Dispositif de détection de gaz selon l'une des revendications 1 à 3, **caractérisé en ce que** le réservoir de gaz (6), la vanne d'arrêt (9) et l'ouverture de sortie de gaz (7a, 7b) sont configurés sous la forme d'unité de test mobile.

5. Dispositif de détection de gaz selon l'une des revendications 1 à 4, **caractérisé en ce qu'**entre le réservoir de gaz (6) et l'ouverture de sortie de gaz (7a, 7b) se trouve un élément de réglage pour régler le débit volumique du gaz témoin et/ou que la vanne d'arrêt (9) est configurée sous la forme d'un élément de réglage pour régler le débit volumique.

6. Dispositif de détection de gaz selon l'une des revendications 1 à 5, **caractérisé en ce que** le dispositif de test présente une mémoire de données (13) dans laquelle est enregistrée, pour chaque détecteur de gaz (3a - 3i), une plage de valeurs de référence pour le temps de propagation nécessaire à la diffusion du gaz témoin de l'ouverture de sortie de gaz (7a, 7b) jusqu'au détecteur de gaz (3a - 3i) concerné, que le dispositif de test présente un dispositif de mesure du temps pour détecter la durée entre l'ouverture de la vanne d'arrêt (9) et la détection du gaz témoin au niveau de chacun des détecteurs de gaz (3a - 3i) et que le dispositif de test présente un dispositif de comparaison pour comparer les durées mesurées avec les plages de valeurs de référence enregistrées et qui est en liaison d'asservissement avec l'indicateur de fonctionnement.

7. Dispositif de détection de gaz selon l'une des revendications 1 à 6, **caractérisé en ce que** dans la mémoire de données (13) est enregistré le tracé dans le temps d'un signal de consigne de test pour l'au moins un détecteur de gaz (3a - 3i) et que le dispositif de test présente un dispositif de comparaison pour comparer le signal de consigne de test avec le tracé dans le temps du signal de mesure (10a - 10f) de l'au moins un détecteur de gaz (3a - 3i).

8. Dispositif de détection de gaz selon l'une des revendications 1 à 7, **caractérisé en ce qu'**il peut être commuté entre un mode de surveillance, dans lequel le dispositif d'interprétation (5) est relié avec l'émetteur d'alarme, et un mode de test dans lequel une sortie du dispositif de comparaison est reliée avec un indicateur de fonctionnement pour l'au moins un détecteur de gaz (3a - 3i).

9. Dispositif de détection de gaz selon l'une des revendications 1 à 8, **caractérisé en ce qu'**au moins trois détecteurs de gaz (3a - 3i) sont prévus dans des espaces (2a, 2b) différents d'un bâtiment (1) ou d'un véhicule, qu'au moins une ouverture de sortie de gaz (7a, 7b) disposée en position fixe par rapport à l'espace (2a, 2b) concerné est à chaque fois prévue dans chacun de ces espaces (2a, 2b), et que le réservoir de gaz (6) est réalisé sous la forme d'un réservoir de gaz (6) central relié avec les ouvertures de sortie de gaz (7a, 7b) par le biais de l'au moins une vanne d'arrêt (9) et d'au moins une conduite de gaz témoin (8).

10. Dispositif de détection de gaz selon l'une des revendications 1 à 9, **caractérisé en ce qu'**il présente un dispositif de commande pour l'au moins une vanne d'arrêt (9), au moyen duquel la vanne d'arrêt (9) peut être commandée de telle sorte qu'un modèle de concentration de gaz témoin différent du modèle de concentration du gaz qui se produit en mode de surveillance est délivré au niveau de l'ouverture de sortie de gaz (7a, 7b), que l'au moins un détecteur de gaz (3a - 3i) présente un dispositif de détection pour détecter le modèle de concentration de gaz témoin et que l'indicateur de fonctionnement est activé lors de la détection du modèle de concentration de gaz témoin.

11. Dispositif de détection de gaz selon l'une des revendications 1 à 10, **caractérisé en ce que** le gaz témoin n'est pas toxique.

12. Dispositif de détection de gaz selon l'une des revendications 1 à 11, **caractérisé en ce qu'**un capteur de température destiné à détecter la température ambiante du détecteur de gaz (3a - 3i) est disposé à côté de chaque détecteur de gaz (3a - 3i).

13. Dispositif de détection de gaz selon l'une des revendications 1 à 12, **caractérisé en ce que** le dispositif de détection de gaz présente un chauffage et/ou un dispositif de refroidissement pour amener le gaz témoin à une température donnée.

14. Dispositif de détection de gaz selon l'une des revendications 1 à 13, **caractérisé en ce que** le gaz témoin est un mélange gazeux qui contient au moins un premier et un deuxième gaz qui présentent une vitesse de diffusion dans l'air différente, qu'au moins un premier détecteur de gaz (3a - 3i) est sensible au premier gaz et au moins un deuxième détecteur de gaz (3a - 3i) au deuxième gaz et que le premier détecteur de gaz (3a - 3i) ainsi que le deuxième détecteur de gaz (3a - 3i) sont disposés dans un boîtier de détection commun.

15. Procédé pour contrôler le fonctionnement d'un dispositif de détection de gaz comprenant au moins deux détecteurs de gaz (3a - 3i) disposés dans au moins un espace à surveiller (2a, 2b), un gaz témoin étant libéré, auquel les détecteurs de gaz (3a - 3i) sont sensibles et le gaz témoin étant détecté à l'aide des détecteurs de gaz (3a - 3i), **caractérisé en ce que** le gaz témoin est libéré à un endroit de l'espace de telle sorte qu'il atteint les au moins deux détecteurs de gaz (3a - 3i) depuis cet endroit et qu'il est détecté par chacun de ces détecteurs de gaz.

16. Procédé selon la revendication 15, **caractérisé en ce que** la durée entre la libération du gaz témoin et la détection du gaz témoin est mesurée et comparée avec une plage de valeurs de référence et qu'un défaut est détecté si un écart est constaté entre la durée mesurée et la plage de valeurs de référence.

17. Procédé selon la revendication 15 ou 16, **caractérisé en ce que** le gaz témoin est libéré en un premier endroit de l'espace à surveiller et il est ensuite détecté à l'aide des détecteurs de gaz, que le gaz témoin est ensuite évacué de l'espace, et que le gaz témoin est ensuite de nouveau libéré en un deuxième endroit de l'espace à surveiller et détecté à l'aide des détecteurs de gaz.

18. Procédé selon l'une des revendications 15 à 17, **caractérisé en ce que** le gaz témoin est un mélange gazeux qui contient au moins deux gaz qui présentent des vitesses de diffusion dans l'air différentes.
